(19) **Europäisches Patentamt**

**European Patent Office**

**Office européen des brevets**

(11) **EP 1 568 674 A1**

(12) **EUROPEAN PATENT APPLICATION**

(43) Date of publication:
**31.08.2005 Bulletin 2005/35**

(51) Int Cl.[7]: **C07C 1/10**, C07C 1/04,
C07C 9/04

(21) Application number: **04003198.1**

(22) Date of filing: **12.02.2004**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(71) Applicant: **PAUL SCHERRER INSTITUT**
**5232 Villigen PSI (CH)**

(72) Inventors:
• **Seemann, Martin**
**5430 Wettingen (CH)**
• **Biollaz, Serge, Dr.**
**4123 Allschwil (CH)**
• **Stucki, Samuel, Dr.**
**5415 Nussbaumen (CH)**

(74) Representative: **Fischer, Michael et al**
**Siemens AG,**
**Postfach 22 16 34**
**80506 München (DE)**

(54) **A process for the synthetic generation of methane**

(57)    The present invention discloses a process for the synthetic generation of methane from a feed gas mixture comprising carbon monoxide, hydrogen and water vapour and optionally aromatic hydrocarbons; said process comprising the steps of:

a) bringing the feed gas mixture in contact with a fluidized bed catalyst having catalyst particles which comprise as catalytic active component a metal and/or a metal compound or a mixture thereof under the circumstances of:
b) an elevated temperature in the range of 250 to 500°C;
c) a feed gas pressure in the range of 0.8 to 70 bar;
d) an gas hourly space velocity of 1000 to 50000 h$^{-1}$; and
e) an concentration of $H_2/CO$ in the initial gas mixture in the range of 0.25 to 5.

The afore-mentioned process allows to catalytically convert hydrogen and carbon monoxide effectively in the fluidized bed catalyst which avoids an advanced deactivation of the catalyst material and therefore delivers a high activity of the catalytic active components in the process. Both thermochemical reactions, the endothermic reformation of higher hydrocarbons, i.e. aromatic hydrocarbons, and the exothermic methane generation, themselves synergetic fertilize which in fact tremendously increases the tolerance of this process against the content of higher hydrocarbons in the feed gas mixture, such a the raw synthesis gas of a wood gasification process, by simultaneously enhancing the robustness of the catalytic activity and selectivity of the catalyst particles.

**Description**

The invention relates to a process for the synthetic generation of methane from a feed gas mixture comprising carbon monoxide, hydrogen and water vapour and optionally aromatic hydrocarbons.

Even there does not exist actually a distinct proof for the greenhouse effect and accordingly the global warming, it is the intention of the world-wide community to reduce the production of gaseous climate relevant components, such as chlorofluorocarbons (CFC) and carbon dioxide. For the reduction of the CFC's significant process has been achieved in the recent years since its production has been worldwide downsized to almost zero. For the carbon dioxide the worldwide community committed itself to reduce the production within the next decade in the range of about 10 to 15%, depending on the country and its commitment to the Kyoto agreement.

For instance, Switzerland committed itself to reduce the production of carbon dioxide originating from traffic related combustion processes by 8% until 2012. Even under an optimistic consideration, it is most unlikely that this ambitious target will be met. As a possible alternative, so-called biofuels have the potential to influence the carbon dioxide balance tremendously on a midterm and long-term basis as being a relevant source for renewable primary products, such as wood or related biomass. The estimated cost are expected in the range of 300 Swiss Francs per ton of $CO_2$. This cost are compared to other options taken in the transportation sector quiet un-expensive.

Beside the effect of the efficient reduction of carbon dioxide the aspect of growing the independency from the crude oil syndicates led to an increasing focus on biofuels. The commission of the EU intents to gain in the year 2020 at least 20% of the consumed fuel from alternative fuels. Beside natural gas (methane) and hydrogen the biofuels are categorized as potential alternative. On a short-term basis fuels like RME (bio diesel), ethyl alcohol and bio gas, are available; in midterm and long-term range, bio methyl alcohol and so-called Fischer-Tropsch fuel shall be produced according to the already theoretically well-known thermo-chemical processes which takes advantage of an catalyst in order to transform a synthesis gas into the desired bio fuel, such as methyl alcohol, gasoline, diesel, methane and hydrogen.

A most promising fuel is methane that is supplied actually as fossil gas and which can be replaced under efficient use of synergies between the required infrastructure for both the fossil gas and the biogen gas by the latter that can be produced by fermentation as well as by thermo-chemical processes.

Unfortunately, one of the most preferred renewable primary product, wood, can not be transformed into bio gas by fermentation. Therefore, it is an crucial task to provide efficient alternative processes for the synthetic generation of methane. Such processes are under intensive R&D activities in the scientific and economical world and some of the process have been revealed as being practicable, even under severe economical considerations.

A process known in the art uses wood in a gasification reactor, such as FICFB (*Fast Internally Circulation Fluidiced Bed),* which requires subsequently non-negligible efforts for the pretreatment of the raw synthesis gas in order to allow both effective and efficient synthesis of methane. As far as wood is considered as the renewable primary product, it has to be pointed out that the raw synthesis gas originating from the gasification step, is loaded with considerable amounts of aromatic hydrocarbons which are known in the prior art to deploy a negative impact in the subsequent process chain. Therefore, the removal of these aromatic hydrocarbons, such as benzene, toluene and naphthaline (BTN), is acknowledged as being a required process requisite prior to a subsequent removal of ammonia $NH_3$ and hydrosulfide $H_2S$. Unfortunately, these additional process requisites tend to increase the cost of the synthetic generation of methane from wood, which is an almost exhaustless renewable primary product and reduces the overall efficency.

For the reason given above, the crucial prerequisite for a commercialized exploitation of wood and other biomasses for the synthetic generation of methane is to find an optimized concert between the processes of gasification, raw synthesis gas purification, methane generation and catalyst properties which is required in the methane generation.

Accordingly, it is the aim of the invention to provide a process which allows the synthetic generation of methane for a renewable primary product, explicitly including wood and related materials, in an economically and commercially reasonable manner.

This aim is achieved according to the invention by a process for the synthetic generation of methane from a feed gas mixture, i.e. a feed gas mixture originating from a biomass gasification process, comprising carbon monoxide, hydrogen and water vapour and optionally aromatic hydrocarbons; said process comprising the steps of:

    a) bringing the feed gas mixture in contact with a fluidized bed catalyst having catalyst particles which comprise as catalytic active component a metal and/or a metal compound or a mixture thereof;
    under the circumstances of:
    b) an elevated temperature in the range of 250 to 500°C;
    c) a feed gas pressure in the range of 0.8 to 70 bar;
    d) an gas hourly space velocity of 1000 to 50000 $h^{-1}$; and
    e) an concentration of $H_2/CO$ in the feed gas mixture in the range of 0.25 to 5.

The afore-mentioned process allows to catalytically convert hydrogen and carbon monoxide effectively in the fluidized bed catalyst which avoids an advanced deactivation of the catalyst material and therefore delivers a high activity of the catalytic active components in the process. Both thermo-chemical reactions, the endothermic reformation of higher hydrocarbons, i.e. aromatic hydrocarbons, and the exothermic methane generation, themselves synergetically fertilize which in fact tremendously increases the tolerance of this process against the content of higher hydrocarbons in the feed gas mixture, such a the raw synthesis gas of a wood gasification process, by simultaneously enhancing the robustness of the catalytic activity of the catalyst particles.

With respect to the initial and continuing catalytic activity, excellent results have been achieved by using as catalytically active component nickel and/or a nickel compound, preferably a mixture of nickel and nickel monoxide, disposed on an ceramic carrier, such as $Al_2O_3$, $TiO_2$, $SiO_2$ or $Y_2O_3$ or mixtures thereof. Additionally, the content of the catalytically active component may be in the range of 20 to 80 weight%, preferably 40 to 60 weight%, as compared to the weight of the catalyst particles. A suitable catalyst may comprise nickel and nickel oxide diposed on alumina ($Al_2O_3$) having a content of the catalystically active component in the range of 50 weight% as calculated as neat nickel.

In order to achieve both a high mobility of the catalyst particles in the fluidized bed catalyst and a sufficient BET surface, the size of the catalyst particles may be in the range of 10 to 1000 $\mu$m, preferably in the range of 100 to 500 $\mu$m.

With respect to the energy balance of the exothermic generation of methane an advantageous feed gas composition is considered to have a positiv impact thereupon when the feed gas mixture comprises aromatic hydrocarbons, such as benzene, toluene and naphthalene, in the range of less than 10 Vol%, preferably less than 5 vol%. Explicitly, this gas composition covers broadly a synthesis gas that originates from wood gasification processes to which a major focus is laid upon due to the environmental demands.

A superior yield with respect to the generation of methane as well as to a complete conversion of the higher hydrocarbons may require that the gas hourly space velocity (GHSV) is in the range of 2000 to 10000 $h^{-1}$, the temperature is in the range of 340 to 400°C and the gas pressure is in the range of 1? Ausführungsbeispiel? bar. With respect to the above-mentioned nickel/nickel oxide catalyst, the temperature is preferably in the range of 350°C, the GHSV in the range of 4200 $h^{-1}$ and the pressure is ambient pressure.

In order to offer ambient condition in the fluidized bed catalyst that supports both the catalytic reactions of reforming higher hydrocarbons and formation of methane and the regeneration of the catalyst particles, a mean residence time of the feed gas mixture in the fluidized bed catalyst may range from 0.1 to 5 sec., preferably 0.2 to 1 sec.

Again with respect to the yield of methane and to the reformation of higher hydrocarbons the content of $H_2/CO$ in the feed gas mixture is in the range of 0.8 to 3, for example in the range of 1.5 as combined with the afore-mentioned nickel/nickel oxide catalyst.

Exemplarily embodiments of the inventive process are described in detail below without the intention to limit the invention to these actually preferred examples.

The measurements were taken with a plant as shown schematically below in Figure 1. The measurement campaign can be divided in three different sections:

Phase 1: The feed gas mixture is pretreated with a washer unit and an activated carbon filter (charcoal absorber);

Phase 2: The feed gas mixture is now only pretreated with the washer unit; and

Phase 3: The feed gas mixture is taken as of the outlet of the wood gasification.

Especially for the intention of maintaining the activity and selectivity of the catalyst, unsaturated hydrocarbons (such as $C_2H_4$, $C_2H_2$), light tar components (such as benzene, toluene, naphthaline, C8: phenylacethylene, styrole, indene) and ammonia are considered to highly disturb these demands.

Figure 1 shows that the complete plant comprise an inlet at 1) for the feed gas mixture originating from a non-illustrated wood gasification unit, followed by a washer unit at 2), an activated carbon filter at 3), a gas pump and the respective unit COALA for the generation of methane which is observed at the outlet of COALA at 4). The methane generation unit COALA comprises a desulphurisation unit in form of a ZnO fixed bed catalyst, an inlet for the supply of water vapour, a fluidized bed catalyst, a filter and a heat exchanger as seen in direction of the gas flow.

In phase 1, the washer unit for the removal of ammonia $NH_3$ and the activated carbon filter for the removal of higher hydrocarbon are disposed upstream the COALA. The gas composition during the first 40 hours of service is given in table 1; the first line giving the data for the gas composition upstream the activated carbon filter, second and third line the respective gas compositions downstream the activated carbon filter after 3 and 40 h of service, resp.

Table 2 gives the gas compositions in phase 1 with respect to benzene, toluene, naphthaline and C8 at the probe locations 1), 2), 3) and 4). Almost all disturbing higher hydrocarbons are already removed at 3) grace to both the washer unit and the activated carbon filter. The plant was operated continuously at a temperature of about 360°C, a water content of 0.25 as related to the dry gas volume and a gas volume flow rate of 0,6 m³/h.

During phase 2, the plant was operated without activated carbon filter and the gas composition with respect to higher hydro carbons is given in table 3.

During phase 3, the plant was operated without any pretreatment of the feed gas mixture. As already before in phases 1 and 2, for the fluidized bed catalyst 100 g catalyst particles having a size of 200 μm and comprising an even content of nickel and nickel oxide supported by alumina ($Al_2O_3$) whereby the content of the catalytically active nickel components is 50 weight% as calculated as neat nickel, was used. The gas composition for the higher hydrocarbons is given in table 4. At the entrance the average load of benzene, toluene, naphthaline and C8 was in the range of 13.6 g/Nm³ resp. 0.6 g/Nm³ resp. 0.8 g/Nm³ resp. 0.5 g/Nm³. At the outlet at 4) these higher hydrocarbons were reformed almost completely without the occurrence of any catalyst deactivation or loss in selectivity.

The comparison data given in table 5 is in so far self-explanatory, when the following definitions are observed:

The steam/dry gas ratio

$$D_V = \frac{n_{H2O}}{n_{Gas\,tr.}}\;[-]$$

and the catalyst load

$$KB = \frac{n_{Gas\,tr}+n_{H2O}}{M_{Kat}}\quad\left[\frac{mol}{kg\cdot h}\right]$$

originating from the generation of methane from synthesis gas, such as pure $H_2$/CO gas. The "steam to carbon ratio"

$$S/C = \frac{n_{H2O}}{n_{Carbon}}\;[-]$$

is known from the steam reforming of methane. All above mentioned key figures are used in a sense that clearly determined distinct gas compositions were used. Therefore, they are not sufficient in order to describe the process of generating methane from a synthesis gas being achieved by a biomass gasification process. Additionally, the H/C ratio in both a dry product gas

$$H/C_{tr} = \frac{n_H}{n_C}$$

as well as in a humidified product gas

$$H/C_f = \frac{n_H + 2\cdot n_{H2O}}{n_C}$$

is a suitable parameter to characterize the process properly. A further meaningful parameter is the conversion rate of carbon monoxide

$$U_{CO} = \frac{n_{COin}-n_{COout}}{n_{Coin}}\;[-],$$

the selectivity to methane

$$S_{CH4} = \frac{CH4in-n_{CH4out}}{n\,(n_{COin}-nC_{Oout})+x\cdot(n_{CxHyin}-n_{CxHyout})}\;[-]$$

and the so called cold gas efficiency,

$$\eta_{KG} = \frac{Heat\;value_{CH4out}}{Heat\;value_{in}}$$

which describes the thermal power at the outlet with respect to the output of methane in relation to the overall thermal power at the inlet.

Surprisingly, the catalyst does not show any effect of deactivation and/or loss in selectivity what is indicated by the data given in the third and forth line. The third line stands for the results in a phase 3a during the initial hours of operation. The results in the forth line stand for the gas compositions that have been achieved by continuously operating the plant over dozends of hours.

**Claims**

1. A process for the synthetic generation of methane from a feed gas mixture comprising carbon monoxide, hydrogen and water vapour and optionally aromatic hydrocarbons; said process comprising the steps of:

   a) bringing the feed gas mixture in contact with a fluidized bed catalyst having catalyst particles which comprise as catalytic active component a metal and/or a metal compound or a mixture thereof under the circumstances of:
   b) an elevated temperature in the range of 250 to 500°C;
   c) a feed gas pressure in the range of 0.8 to 70 bar;
   d) an gas hourly space velocity of 1000 to 50000 h$^{-1}$; and

e) an concentration of $H_2/CO$ in the initial gas mixture in the range of 0.25 to 5.

2. The process according to claim 1, **characterized in that** the catalytic active component is nickel and/or a nickel compound, preferably a mixture of nickel and nickel oxide, disposed on an ceramic carrier, such as $Al_2O_3$, $TiO_2$, $SiO_2$ or $Y_2O_3$ or mixtures thereof.

3. The process according to claim 2, **characterized in that** the content of the catalytically active component is in the range of 20 to 80 weight%, preferably 40 to 60 weight%, as compared to the weight of the catalyst particles.

4. The process according to claim 1, 2 or 3, **characterized in that** the size of the catalyst particles is in the range of 10 to 1000 $\mu$m, preferably in the range of 100 to 500 $\mu$m.

5. The process according to anyone of the preceding claims, **characterized in that** the feed gas mixture comprises aromatic hydrocarbons, such as benzene, toluene and naphthalene, in the range of less than 10 Vol%, preferably less than 5 vol%.

6. The process according to anyone of the preceding claims, **characterized in that** the gas hourly space velocity is in the range of 2000 to 10000 $h^{-1}$, the temperature is in the range of 340 to 400°C and the gas pressure is in the range of 1 bar.

7. The process according to anyone of the preceding claims, **characterized in that** a mean residence time of the feed gas mixture in the fluidized bed catalyst is in the range of 0.1 to 5 sec., preferably 0.2 to 1 sec.

8. The process according to anyone of the preceding claims, **characterized in that** the content of $H_2/CO$ in the feed gas mixture is in the range of 0.8 to 2.

**EP 1 568 674 A1**

**European Patent Office**

## EUROPEAN SEARCH REPORT

Application Number

EP 04 00 3198

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 011 058 A (TABLER DONALD C ET AL) 8 March 1977 (1977-03-08) *abstract; columns 2-7 and the claims* ----- | 1-8 | C07C1/10 C07C1/04 C07C9/04 |
| X | US 3 600 145 A (JOHNSON MARVIN M ET AL) 17 August 1971 (1971-08-17) * the whole document * ----- | 1-8 | |
| X | EP 0 086 538 A (VEG GASINSTITUUT NV) 24 August 1983 (1983-08-24) *abstract; pages 2, 3, 6, 11-14, 24; the examples and the claims* ----- | 1-8 | |
| X | US 3 927 999 A (ROBIN ALLEN M ET AL) 23 December 1975 (1975-12-23) * the whole document * ----- | 1-8 | |

TECHNICAL FIELDS SEARCHED (Int.Cl.7)

C07C

The present search report has been drawn up for all claims

| Place of search | Date of completion of the search | Examiner |
|---|---|---|
| Munich | 9 August 2004 | Lorenzo Varela, M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 00 3198

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2004

| Patent document cited in search report | | Publication date | Patent family member(s) | | Publication date |
|---|---|---|---|---|---|
| US 4011058 | A | 08-03-1977 | NONE | | |
| US 3600145 | A | 17-08-1971 | NONE | | |
| EP 0086538 | A | 24-08-1983 | NL | 8200544 A | 01-09-1983 |
| | | | AU | 560415 B2 | 09-04-1987 |
| | | | AU | 1130783 A | 18-08-1983 |
| | | | DE | 3360491 D1 | 12-09-1985 |
| | | | EP | 0086538 A1 | 24-08-1983 |
| | | | JP | 58148829 A | 05-09-1983 |
| | | | ZA | 8300920 A | 30-11-1983 |
| US 3927999 | A | 23-12-1975 | DE | 2460792 A1 | 10-07-1975 |
| | | | ES | 433278 A1 | 16-11-1976 |
| | | | FR | 2256234 A1 | 25-07-1975 |
| | | | IT | 1028081 B | 30-01-1979 |
| | | | JP | 50098505 A | 05-08-1975 |
| | | | ZA | 7407700 A | 28-04-1976 |

EPO FORM P0459

For more details about this annex : see Official Journal of the European Patent Office, No. 12/82